Europäisches Patentamt

(19) **European Patent Office**

Office européen des brevets

(11) Publication number: **0 027 730**
**A2**

(12) # EUROPEAN PATENT APPLICATION

(21) Application number: **80303678.9**

(22) Date of filing: **17.10.80**

(51) Int. Cl.³: **A 61 K 7/00**
**A 61 K 7/48**

(30) Priority: **17.10.79 JP 132872/79**
**27.11.79 JP 152483/79**

(43) Date of publication of application:
**29.04.81 Bulletin 81/17**

(84) Designated Contracting States:
**AT BE CH DE FR GB IT LI LU NL SE**

(71) Applicant: **JOHNSON COMPANY, LIMITED**
**699-1, Aza-Kitagawa Kokufu-Hongo**
**Oisomachi Naka-gun Kanagawa-ken(JP)**

(72) Inventor: **Kitamura, Rihachi**
**445-2, Nakamurahara**
**Odawara-shi Kanagawa-ken(JP)**

(72) Inventor: **Ujike, Toshiaki**
**480-1, Nakamurahara**
**Odawara-shi Kanagawa-ken(JP)**

(74) Representative: **Baillie, Iain C. et al,**
**c/o Langner Parry Blumenstrasse 48**
**D-8000 München 2(DE)**

(54) **A cosmetic composition.**

(57) A cosmetic composition comprises a compound capable of generating heat to raise the temperature of the composition when the compound is brought into contact with water.

The cosmetic composition is used for hair shampoo, hair rinse, hair lotion, hair treatment, hand cleaner, hand lotion, cleansing lotion and moisturizing cream.

EP 0 027 730 A2

∧

TITLE OF THE INVENTION

A Cosmetic Composition


BACKGROUND OF THE INVENTION

Field of the Invention

This invention relates to a cosmetic composition. More particularly, the present invention relates to a cosmetic composition which generates adequate heat when it is used.

Description of the Prior Art

As cosmetic compositions which are usable for hair washing such as shampoo or rinse, for hair such as rinse (conditioner), lotion or hair treatment, and for skin such as hand cleaner or hand cream are cited, and they contain soap and surface active agents as main components, and also conditioners such as oily components, perfumes, dyes, thickners or cationated celluloses, protein derivatives, lanolin derivatives and extracts of natural substance.

Further conditioning cosmetic compositions for hair may contain oily components such as vegetable oil, fatty acid ester, higher alcohol, etc., surface active agents, oil soluble poly(alkylene oxides) derivatives, lanolin derivatives, vegetable components, etc., as main components and also perfumes, dyes, thickners, etc.

A cosmetic composition shows its finishing effect when it is used with water, and its cleansing effect and

- 1 -

finishing effect are enhanced and more comfortable hot feeling is given when it is used with hot water than when it is used with cold water. But it cannot be said that hot water is always arranged.

As conditioning cosmetic compositions for hair, hair washing type rinse, lotion, treatment, etc. may be cited, and their cosmetic effect are shown when they are used with water. In this case, finishing effect is better and more comfortable hot feeling is given when hot water is used rather than when cold water is used. But, it cannot be said that hot water is always arranged.

The inventors of this patent repeated investigations under above circumstance with the object to get a cosmetic composition showing both excellent cleansing effect and excellent finishing effect even if it was used with cold water and then recognized that the object could be easily achieved if such substance that reacts with water to generate heat is used in cosmetic. Therefore, this invention is based on this recognition.

SUMMARY OF THE INVENTION

It is an object of the present invention to provide a cosmetic composition showing excellent cleansing effect even if it was used with cold water.

It is another object of the present invention to provide a cosmetic composition showing excellent finishing effect even if it was used with cold water.

It is a further object of the present invention to provide a cosmetic composition for hair showing excellent finishing effect even if cold water was used.

According to one aspect of the present invention, there is provided a cosmetic composition which comprises a compound capable of generating heat to raise the temperature of the composition when the compound is brought into contact with water.

According to another aspect of the present invention, there is provided a cosmetic composition comprising a member selected from the group consisting of poly(ethylene oxides), poly(propylene oxides) and derivatives thereof and a member of alkylene glycols having 2 to 9 carbon atoms are contained as the compound.

## DESCRIPTION OF THE PREFERRED EMBODIMENTS

A feature of the present invention is to contain such substance which generates heat when it is contacted with water in a cosmetic composition such as hair shampoo, hair rinse, lotion, hair treatment, hand cleaner, etc.

The expression "cosmetic composition" as used in the present invention comprehends a cosmetic composition usable for hair washing, hair treatment, skin, etc. as mentioned above,

4

and the cosmetic composition contains the same components as a conventional general composition. That is, the composition contains soap and/or surface active agents as main components, also perfume, dye, thickner, conditioner, etc. but hydroxy-propyl cellulose is particularly used in the present invention suitably as a thickner.

The present invention comprises containing substance which generates heat of dilution, heat of dissolution, or heat of reaction when it is contacted with water. If the quantity of generated heat is too small, the merit of the present invention cannot be achieved, and if the quantity of generated heat is too large, the composition becomes dangerous and cannot be used safely on the contrary. Generally, such substance is used which raise the temperature thereof up to three or more degrees, preferably up to five or more degrees and further more preferably up to eight or more degrees when the said composition is mixed with cold water in an equal weight ratio. And such substance which raises the temperature thereof up to twenty or more degrees and particularly, up to thirty or more degrees is not preferable in view of safety.

Examples of the substances used in the present invention which generate heat when it is contacted with water are as follows. Alkyl alcohols having 2 to 4 carbon atoms such as ethanol, isopropyl alcohol and the like; alkylene glycols having 2 to 9 carbon atoms such as ethylene glycol, propylene glycol, butylene glycol, pentylene glycol, hexylene glycol and the like; aliphatic triols such as glycerol; di(alkylene glycols) and derivatives thereof having 2 to 4 carbon atoms in the alkylene portion such as di(ethylene glycol), di(propylene glycol), di(ethylene glycol) mono methyl ether, and di(ethylene glycol) mono ethyl ether; and poly (alkylene oxides) (also called poly(oxyalkylene), poly(alkylene glycol), or polyether glycol) and derivatives thereof having 2 to 4 carbon atoms in the alkylene portion such as poly(ethylene glycols), poly(propylene glycols), block or random copolymers of ethylene oxide and propylene oxide, and others of poly (alkylene oxides) having 1 to 5 carbon atoms in the ether portion, for example, butyl mono ethers of copolymers of ethylene oxide and propylene oxide, butyl mono ethers of poly(ethylene oxides), poly(ethylene oxide-propylene oxide) adducts of glycerol, and poly(propylene oxides) adducts of glycerol.

As said alkyl alcohols, it is preferable to use ethanol or isopyl alcohol.

As alkylene glycols having 2 to 9 carbon atoms, alkylene glycol having 6 or less carbon atoms per molecule can be preferably used. But, alkylene glycol having 10 or more carbon atoms per molecule cannot be used in the present invention. It is more preferable to use ethylene glycol, propylene glycol,

6

or hexylene glycol.

As said aliphatic triols, it is preferable to use glycerol.

As said di(alkylene glycols), it is preferable to use di(ethylene glycol) or di(propylene glycol). As said derivatives thereof, it is preferable to use di(ethylene glycol) mono methyl ether or di(ethylene glycol) mono ethyl ether.

As said poly(alkylene oxides), it is preferable to use poly(ethylene glycol)-200, -300, -400, and -600 (said figures represent average molecular weight of the poly(ethylene glycol)), poly(propylene glycols) having 200 to 1200 of average molecular weight or block copolymers of ethylene oxide and propylene oxide.

As said derivatives of said poly(alkylene oxides), it is preferable to use mono butyl ethers of copolymers of ethylene oxide and propylene oxide, poly(ethylene oxide - propylene oxide) adducts of glycerol or poly(propylene oxides) adducts of glycerol.

The present inventors studies widely with the object to obtain such conditioning cosmetic composition for hair showing excellent finishing effect even if cold water was used, and, as the result, they realized a method to use polyalkylene oxides or their derivatives which generated heat when they were contacted with water.

However, poly(alkylene oxides) or derivatives thereof change to oil soluble from water soluble as the number of carbon atoms in alkylene portion increase, and so poly(ethylene oxides), poly(propylene oxides) or derivatives thereof only can actually be used among poly(alkylene oxides) or their derivatives.   On the other hand, since a conditioning cosmetic composition for hair contains oily components such as vegetable oil, mineral oil, fatty acid ester, higher alcohol, oily poly(alkylene oxides) derivatives, that are, for examples, poly(propylene oxides) butylether, poly(propylene glycols), etc., such inconvenience will be resulted that the oily components become insoluble if water soluble poly(ethylene oxides), poly(propylene oxides) or derivatives thereof are used as heat generating components.

The inventors of this invention studied further about this problem and recognized unexpectedly that this problem could be solved easily by using poly(ethylene oxides), poly (propylene oxides) or derivatives thereof along with alkylene glycol having 2 to 9 carbon atoms, which make oil soluble and they completed the present invention.

The substance which generates heat is generally employed in an amount more than 50% by weight and preferably in an amount more than 60% by weight of the total cosmetic composition.   If the ratio of the said substance in the cosmetic composition increases, accordingly the ratio of cleaner decreases, and, generally, the said substance is employed in an amount under 95% by weight, further preferably in an amount under 80% by weight, and more preferably in an amount of 70% by weight or more based on the total cosmetic composition. If the amount is less than 50% by weight, satisfactory heat generating effect is not achieved.

It is preferable that the cosmetic composition for hair according to the present invention contains both poly(ethylene oxides), poly(propylene oxides) or derivatives thereof which generate heat on contact with water and also alkylene glycol having 2 to 9 carbon atoms.

In case of the cosmetic composition for hair, an amount of poly(ethylene oxides), poly(propylene oxides) or derivatives thereof used in this invention is generally 50% by weight or more, preferably, 60% by weight of more, and further preferably 70% by weight or more of the total cosmetic composition. If the amount is less than 50% by weight, satisfactory heat generating effect cannot be achieved. If the amount is too large, the ratio of the essential cosmetic component decreases, and, generally, they are employed in an amount 90% by weight or less and preferably in an amount 80% by weight or less of the said cosmetic composition. Alkylene glycol having 2 to 9 carbon atoms is generally employed in 1.0 to 0.1 weight ratio and preferably in 0.5 to 0.25 weight ratio to poly(ethylene oxides), poly(propylene oxides) or derivatives thereof. Also, hydroxypropyl cellulose is preferably used as a thickner in the cosmetic composition for hair of the present invention.

The cosmetic composition of the present invention can be used to be mixed in a suitable ratio with cold water, and the heat generated by mixing can be controlled by increase or decrease in quantity of water. Foaming and permeability of the cleaner are improved and more excellent cleaning effect can be achieved by generation of heat even if cold water is used. The composition of this invention can be used suitably as a composition for hair since the composition can achieve particularly well balanced oil/water finishes. And also, even though a hair treatment on the market requires an operation of hair steaming with a towel dipped in hot water, the hair treatment of the present invention gives comfortable hot feeling effect and steaming effect can be achieved since the hair treatment generates heat and temperature increase can be achieved by mixing with water existing between hairs after shampoo.

The present invention will be described more concretely by examples, but the present invention shall not be limited to these examples unless exceeding the essential summary of the present invention. In the following examples, all the terms "part" denote part by weight and every chemicals used in the examples meets the Standard of the Raw Material for the Cosmetic Composition unless especially stated.

EXAMPLE 1

Poly(ethylene glycol)-400 (64.5 parts), 10 parts of monooleic acid poly(oxyethylene) sorbitan (20 mol of ethylene oxide), 15 parts of 2-alkyl-N-carboxyethyl-N-hydroxyethyl imidazolium betaine (Softazolin SF supplied by Kawaken-Fine Chemicals), 5 parts of coconut fatty acid diethanolamide, 5 parts of propylene glycol and 0.5 parts of perfume were uniformly mixed with agitation. An adequate quantity of the hair shampoo thus obtained was taken in a hand and then applied directly or after being diluted with water in an amount less than approximately one half quantity of the hair shampoo to hair and head skin which had been wetted with cold water or hot water to conduct the shampooing. As a result, comfortable hot feeling effect was achieved, and finishing effect was good.

EXAMPLE 2

Poly(ethylene glycol)-400 (59.0 parts), 3.0 parts of isopropyl myristate, 0.05 parts of L-menthol, 5.0 parts of hexyl decanol, 1.5 parts of hydroxypropyl cellulose, 1.0 part of hydrolyzed animal protein derivative (Promois E 118D supplied by Seiwa Kasei Co.), 2.0 parts of cetyl trimethyl ammonium chloride (Lepon TM 16 supplied by Sanyo Kasei Co.) and 13.0 parts of hexylene glycol were mixed with agitation. To the resulting mixture was added a solution of 0.005 parts of Orange 205 (dye approved by law) dissolved in 1.0 part of water. Then, a solution of 1.0 part of distearyl dimethyl ammonium chloride dissolved in 12.9 parts of the above mentioned hexylene glycol with heating at 60°C was further added to obtain a hair treatment.

An adequate quantity of the hair treatment was taken in a hand and diluted with cold water in an amount less than about one half quantity of the hair treatment. The diluted hair treatment was then applied to hair which has been rinsed with the shampoo obtained in Example 1 and to head skin so as to rub the hair treatment on them. As a result, hot feeling was given and finishing effect was good.

EXAMPLE 3

Glycerol (30.0 parts), 55.0 parts of propylene glycol, 5.0 parts of 2-hexyl decanol, 5.0 parts of ethanol (99%), 1.0 part of cetyl acetate, 2.0 parts of silicone oil (Toray Silicone SH 3771 oil supplied by Toray Silicone Co.), 2.0 parts of hydrolyzed animal protein derivative (Promoise E 118D supplied by Seiwa Kasei Co.), 0.0001 part of dyestuff and 0.2 parts of perfume were successively mixed uniformly to obtain a hand lotion in a liquid form.

An adequate quantity of the hand lotion was taken in a hand, mixed with water in substantially the same amount as that of the hand lotion uniformly and spread over the hand. As a result, comfortable hot feeling was given due to heat generation upon hydration of the lotion. Further, circulation

/12/

of the bload was facilitated and simultaneously good balance of the moisture content and oil content in the skin was achieved so that the hand skin became smooth.  Also, when the propylene glycol was replaced by ethanol, similar results were obtained.

EXAMPLE 4

Poly(ethylene glycol)-300 (30.0 parts), 30.0 parts of poly(ethylene glycol)-400, 15.0 parts of poly(oxyethylene) (20) monooleate, 15.0 parts of 2-alkyl-N-carboxyethyl-N-hydroxyethyl imidazolium betain (Softazolin SF supplied by Kawaken-Fine Chemicals), 5.0 parts of coconut fatty acid diethanolamide, 0.5 parts of perfume, 0.5 parts of white pigment (Natural Pearlessence AH-1 supplied by Kakuhachi Co., Ltd.) and 4.0 parts of poly(oxyethylene)-poly(oxypropylene) block polymer (Pluronic L 61 supplied by BASF) were successively mixed uniformly to obtain a liquid hand soap.

An adequate quantity of the hand soap was taken in a hand and mixed with cold water in about equivalent amount to that of the soap.  The hand was then washed with the soap. As a result, owing to heat generation upon hydration of the soap, cold feeling due to the cold water was eliminated, and circulation of the blood was improved, and further the moisture content was well balanced so that the skin became smooth.

EXAMPLE 5

Poly(oxyethylene)-poly(oxypropylene) block polymer (10.0 parts), 56.6 parts of propylene glycol, 20.0 parts of ethanol, 5.0 parts of 2-alkyl-N-carboxyethyl-N-hydroxyethyl imidazolinum betain (Softazolin SF supplied by Kawaken-Fine Chemicals), 0.2 parts of isopropyl myristate, 0.2 parts of 2-hexyldecanol and 3.0 parts of poly(oxyethylene)(20) mono-oleate were successively mixed uniformly to obtain a liquid cleansing lotion.

An adequate quantity of the cleansing lotion was mixed with water. Then, a cut cotton was wetted with the lotion and used to wash off the make-up, and thereafter the face was washed with water. As a result, owing to heat generation upon hydration of the lotion, the activation was enhanced and the cleansing effect was improved.

EXAMPLE 6

Propylene glycol (30.4 parts), 25.0 parts of ethanol, 5.0 parts of zinc chloride, 32.0 parts of poly(ethylene glycol) -300, 5.0 parts of 2-hexyl decanol, 2.0 parts of hydrolyzed animal protein derivative (Promois E 118 D supplied by Seiwa Kasei Co.), 0.1 part of purified water and 0.5 parts of perfume were successively mixed uniformly to obtain a hot shower oil in a liquid form.

Small portions of the product were taken in a hand, applied to and spread over skin which had been wetted after shower. Thereafter, the skin was slightly showered. In this case, circulation of the blood was improved by heat generation due to hydration of the product with the moisture content on the skin, and non-greasy and smooth feeling was given.

EXAMPLE 7

1,3-Butylene glycol (21.2 parts), 30.0 parts of poly(ethylene glycol)-600 and 25.0 parts of propylene glycol were mixed, to which hydroxypropyl cellulose was added, and the mixture was further mixed with heating at 80 - 85°C. Under this temperature condition, ethylene glycol distearate was added, and the resulting mixture was then cooled to 40 - 45°C. Further, 15.0 parts of ethanol and 3.0 parts of 2-hexyl decanol were added. After the mixture was allowed to stand at room temperatures, 2.5 parts of isopropyl myristate, 0.2 parts of white pigment (Natural Pearlessence AH-1 supplied by Kakuhachi Co., Ltd.) and 0.1 part of purified water were further added to the mixture and uniformly mixed to obtain a moisturizing cream in a paste form.

An adequate quantity of the cream was taken in a hand, applied to and spread over skin which had been previously wetted with water, in a manner of massage. The skin was then slightly rinsed with water. In this case, comfortable hot feeling was given, and circulation of the blood was facilitated, and further good balance of the moisture content and oil content was achieved so that the skin became smooth.

15

EXAMPLE 8

While poly(ethylene glycol)-300 (58.50 parts) and 26.45 parts of hexylene glycol were agitated in a batch, 1.50 parts of hydroxypropyl cellulose was added, and the mixture was then completely dissolved by heating at 80 - 85°C. 4.00 parts of cetyl trimethyl ammonium chloride (Lepon TM 16 supplied by Sanyo Kasei Co.) was added to and dissolved in the solution which was being cooled. Further, 3.00 parts of isopropyl myristate, 5.00 parts of oleyl alcohol and 1.00 part of hydrolyzed animal protein derivative (Promois E 118D supplied by Seiwa Kasei Co.) were added and made uniform. The resultant was allowed to stand at 45°C, and 0.05 parts of menthol, 0.50 parts of perfume (JN 333 supplied by Hasegawa Koryo K.K.) and an aqueous solution of an adequate amount of coloring matter (Yellow 4, Blue 1) in 0.10 part of purified water was added and made uniform to obtain a hair treatment. Trial tests of this hair treatment were conducted by standard methods applying it to ten housewives. As a result, the ten housewives observed that the hair treatment did not require steaming operation due to adequate heat generation and it was less sticky than the conventional cream type product and further showed moist and supple finishing effect.

EXAMPLE 9

A hair treatment was prepared by using the same recipe as in Example 8 except that the poly (ethylene glycol) was

- 15 -

16

replaced by 50.00 parts of poly(oxyethylene)-poly(oxypropylene) butylether and the hexylene glycol alone was replaced by 20.00 parts of hexylene glycol and 19.40 parts of 1,3-butylene glycol in combination and further the oleyl alcohol was replaced by 3.00 parts of hexyl decanol. This hair treatment showed the same finishing effect as that obtained by the product of Example 8.

EXAMPLE 10

Poly(oxyethylene)-poly(oxypropylene) butylether (44.2 parts), 20.0 parts of poly(ethylene glycol)-300, 25.0 parts of hexylene glycol, 1.0 parts of camellia oil, 3.0 parts of isopropyl nyristate, 4.0 parts of 2-hexyl decanol, 1.2 parts of behenyl trimethyl ammonium chloride, 1.0 parts of hydrolyzed animal protein derivative (Promois E 118D supplied by Seiwa Kasei Co.), 0.5 parts of perfume and 0.1 part of purified water were successively mixed uniformly to obtain a hair oil rinse in a liquid form.

An adequate quantity of the hair oil rinse was applied to hair which had been shampooed, and uniformly spread in a manner of massage together with water in an about equivalent amount to that of the oil rinse. The hair was then washed with water. As a result, hydration of the oil rinse spontaneously generated heat, gave comfortable hot feeling effect and improved circulation of the blood in the root of the hair.

WHAT WE CLAIM IS:

1. A cosmetic composition which comprises a compound capable of generating heat to raise the temperature of the composition when the compound is brought into contact with water.

2. A cosmetic composition according to Claim 1 in which the compound causes a temperature rise of not less than 3°C when the compound is mixed with an equivalent weight of water.

3. A cosmetic composition according to Claim 1 or 2 in which the compound is contained in an amount of 50 - 95% by weight based on the total cosmetic composition.

4. A cosmetic composition according to Claim 1 or 2 in which the compound is a member selected from the group consisting of alkyl alcohols having 2 to 4 carbon atoms, alkylene glycols having 2 to 9 carbon atoms in the alkylene portion, glycerol, di(alkylene glycols) and derivatives thereof having 2 to 4 carbon atoms in the alkylene portion, and poly (alkylene oxides) and derivatives thereof having 2 to 4 carbon atoms in the alkylene portion.

5. A cosmetic composition according to Claim 4, in which the derivatives of the di(alkylene glycols) are alkyl mono ethers of the di(alkylene glycols) having 1 to 5 carbon atoms in the alkyl portion.

6. A cosmetic composition according to Claim 4, in which the derivatives of the poly(alkylene oxides) are alkyl mono ethers of the poly(alkylene oxides) having 1 to 5 carbon atoms in the alkyl portion.

7. A cosmetic composition according to Claim 4, in which the derivatives of the poly(alkylene oxides) are the poly (alkylene oxide) adducts of glycerol.

8. A cosmetic composition according to Claim 1 or 2 in which both a member selected from the group consisting of poly (ethylene oxides), poly(propylene oxides) and derivatives thereof and a member of alkylene glycols having 2 to 9 carbon atoms are contained as the compound.

9. A cosmetic composition according to Claim 8 in which the former member is contained in an amount of 50 - 90% by weight based on the total cosmetic composition and the latter member is contained in an amount of 0.1 - 1.0 part by weight per one part by weight of the former member, the sum of the former member and the latter member being not more than 100%.

10. A cosmetic composition according to Claim 8, in which the derivatives of the poly(ethylene oxides) and poly(propylene oxides) are alkyl mono ethers of the poly(ethylene oxides) and poly(propylene oxides) having 1 to 5 carbon atoms in the alkyl portion.

11. A cosmetic composition according to Claim 8, in which the derivatives of the poly(ethylene oxides) and poly(propylene oxides) are poly(ethylene oxides)-, poly(propylene oxides)- or poly(ethylene oxide and propylene oxide)- adducts of glycerol.

12. A cosmetic composition according to Claim 8 or 9 which is used as a conditioning cosmetic composition for hair.

13. A cosmetic composition according to Cliam 1, 2, 3 or 4 which is used for hair shampoo, hair rinse, hair treatment or hand cleaner.